# FASCICULE DE BREVET EUROPEEN

(11) **EP 1 575 960 B1**
(45) Date de publication et mention de la délivrance du brevet: **18.03.2009**
(21) Numéro de dépôt: 03775474.4
(22) Date de dépôt: 06.10.2003
(51) Int. Cl.: C07D 487/08, C07D 519/00, A61K 31/55, A61K 31/551

(54) **DERIVES DE 1,4-DIAZABICYCLO¬3.2.2 NONANECARBOXAMIDES, LEUR PREPARATION ET LEUR APPLICATION EN THERAPEUTIQUE AYANT TRAIT AU DYSFONCTIONNEMENT DES RECEPTEURS NICOTINIQUES**
1,4-DIAZABICYCLO¬3.2.2 NONANECARBOXAMIDES DERIVATE, DEREN HERSTELLUNG, UND DEREN THERAPEUTISCHE VERWENDUNG IM ZUSAMMENHANG MIT NIKOTINISCHEN REZEPTOR-STÖRUNGEN
1,4-DIAZABICYCLO 3.2.2 NONANECARBOXAMIDE DERIVATIVES, PREPARATION AND THERAPEUTIC USE THEREOF

(30) Priorité: 08.10.2002 FR 0212500
(43) Date de publication de la demande: 21.09.2005
(73) Titulaire: Sanofi-Aventis, 75013 Paris (FR)
(72) Inventeur: GALLI, Frédéric, FR-92420 Vaucresson (FR); LARDENOIS, Patrick, F-92340 Bourg-La-Reine (FR); LECLERC, Odile, F-91300 Massy (FR); LOCHEAD, Alistair, F-94220 Charenton (FR)
(74) Mandataire: Gaslonde, Aude
(86) Numéro de dépôt international: PCT/FR2003/002929
(87) Numéro de publication internationale: WO 2004/033456

(56) Documents cités:
- EP-A- 0 307 140
- EP-A- 1 219 622
- WO-A-00/34279
- WO-A-01/55150
- WO-A-01/92261

## Description

Les composés de la présente invention sont des ligands des récepteurs nicotiniques. Ces composés sont utiles dans le traitement ou la prévention des désordres liés à un dysfonctionnement de ces récepteurs, notamment au niveau du système nerveux central.
Les documents WO01/92261, WO01/55150 et WO00/34279 décrivent des dérivés de 1, 4-diazabicyclo [3.2.2] nonane en tant que ligands des récepteurs nicotiniques.
Le document EP1219622 décrit des dérivés diazabicycliques en tant qu'agonistes aux récepteurs nicotiniques de type alpha 7.
Le document EP0307140 décrit des dérivés d'oxadiazolyl-azabicycloheptanes ayant une affinité pour les récepteurs muscariniques.

Les composés de l'invention répondent à la formule générale (I) 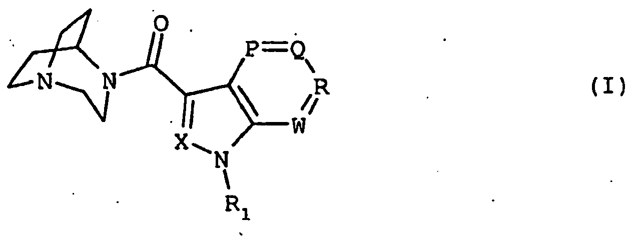 dans laquelle
X représente un atome d'azote ou un groupe de formule générale C-R₂.
P représente un groupe de formule générale C-R_{3;}
Q représente un groupe de formule générale C-R_{4;}
R représente un groupe de formule générale C-R_{5;}
W représente un groupe de formule générale C-R_{6;}
ou bien l'un des symboles P, Q, R et W représente un atome d'azote,
R₁ représente un atome d'hydrogène ou un groupe (C₁-C₆)alkyle,
R₂ représente un atome d'hydrogène ou un groupe (C₁-C₆)alkyle,
R₃, R₄, R₅ et R₆ représentent chacun, indépendamment l'un de l'autre, un atome d' hydrogène ou d'halogène, ou un groupe (C₁-C₆)alkyle, (C₁-C₆)alcoxy, nitro, amino, trifluorométhyle, cyano, ou de formule générale -NR₇R₈, -NR₇C(=O)R₈,
-NR₇C(=O)NR₈R₉, -NR₇C(=O)OR₈, -NR₇S(=O)₂NR₈R₉, -OR₇, -OC(=O)R₇, -OC(=O)OR₇, -OC(=O)ONR₇R₈, -OC(=O)SR₇, -OS(=O)₂R₇, -C(=O)OR₇, -C(=O)R₇, -C(=O)NR₇R₈, -SR₇, -S(=O)R₇, -S(=O)₂R₇, -S(=O)₂NR₇R₈, (C₆-C₁₁) aryle ou (C₃-C₁₂)hétéroaryle éventuellement substitué par un ou plusieurs groupes choisis parmi les atomes d' halogènes, les groupes (C₁-C₆)alkyles, (C₁-C₆)alcoxy, nitro, amino, trifluorométhyles, cyano, ou de formules générales -NR₇R₈ ou -OR₇,

R₇, R₈, et R₉ représentent chacun, indépendamment l'un de l'autre un atome d'hydrogène ou un groupe (C₁-C₆)alkyle linéaire ou branché, un groupe (C₂-C₆)alcényle linéaire ou ramifié, ou un groupe (C₂-C₆)alcynyle linéaire ou ramifié ou un groupe (C₃-C₈)cycloalkyle, (C₄-C₈)cycloalcényle, ou un groupe hétérocycloalkyle ayant 3 à 8 atomes dans le cycle ou un groupe (C₅-C₁₁)bicycloalkyle, (C₇-C₁₁)bicycloalcényle, hétérobicycloalkyle ayant 5 à 11 atomes dans le cycle, bicyclohétéroalcényle ayant 5 à 11 atomes dans le cycle, (C₆-C₁₁)aryle ou hétéroaryle ayant 5 à 12 atomes dans le cycle,

R₇, R₈ et R₉ peuvent être éventuellement substitués, indépendamment l'un de l'autre, par un atome d'halogène, ou un groupe (C₁-C₆)alkyle, (C₁-C₆)alcoxy, nitro, amino, trifluorométhyle, cyano, ou de formule générale -NR₁₀R₁₁, -NR₁₀C(=O)R₁₁, -NR₁₀C(=O)NR₁₁R₁₂, -NR₁₀C(=O)OR₁₁, -NR₁₀S(=O)₂NR₁₁R₁₂, -OR₁₀, -OC(=O)R₁₀, -OC(=O)OR₁₀, -OC(=O)ONR₁₀R₁₁, -OC(=O)SR₁₀, -C(=O)OR₁₀, -C(=O)R₁₀, -C(=O)NR₁₀R₁₁, -SR₁₀, -S(=O)R₁₀, -S(=O)₂R₁₀, -S(=O)₂NR₁₀R₁₁,

R₁₀, R₁₁ et R₁₂ représentent chacun, indépendamment l'un de l'autre, un atome d'hydrogène ou un groupe (C₁-C₆)alkyle linéaire ou ramifié, un groupe (C₂-C₆)alcényle linéaire ou ramifié, ou un groupe (C₂-C₆)alcynyle linéaire ou ramifié ou un groupe (C₃-C₈)cycloalkyle, (C₄-C₈)cycloalcényle, ou un groupe hétérocycloalkyle ayant 3 à 8 atomes dans le cycle ou un groupe (C₅-C₁₁)bicycloalkyle, (C₇-C₁₁)bicycloalcényle, hétérobicycloalkyle ayant 5 à 11 atomes dans le cycle, bicyclohétéroalcényle ayant 5 à 11 atomes dans le cycle, (C₆-C₁₁)aryle ou hétéroaryle ayant 5 à 12 atomes dans le cycle,

R₁₀, R₁₁ et R₁₂ peuvent être éventuellement substitués, indépendamment l'un de l'autre, par un atome d'halogène, ou un groupe (C₁-C₆)alkyle, (C₁-C₆)alcoxy, nitro, amino, trifluorométhyle, cyano, ou -NR₁₃R₁₄, -NR₁₃C(=O)R₁₄, -NR₁₃C(=O)NR₁₄R₁₅. -NR₁₃C(=O)OR₁₄, -NR₁₃S(=O)₂NR₁₄R₁₅, -OR₁₃, -OC(=O)R₁₃, -OC(=O)OR₁₃, -OC(=O)ONR₁₃R₁₄, -OC(=O)SR₁₃, -C(=O)OR₁₃, -C(=O)R₁₃, -C(=O)NR₁₃R₁₄, -SR₁₃, -S(=O)R₁₃, -S(=O)₂R₁₃, -S(=O)₂NR₁₃R₁₄,

R₁₃, R₁₄ et R₁₅ représentent chacun, indépendamment l'un de l'autre, un atome d'hydrogène ou un groupe (C₁-C₆)alkyle linéaire ou ramifié, un groupe (C₂-C₆)alcényle linéaire ou ramifié, ou un groupe (C₂-C₆)alcynyle linéaire ou ramifié,
ou un groupe (C₃-C₈)cycloalkyle, (C₄-C₈)cycloalcényle, ou un groupe hétérocycloalkyle ayant 3 à 8 atomes dans le cycle,
ou un groupe (C₅-C₁₁)bicycloalkyle, (C₇-C₁₁)bicycloalcényle, hétérobicycloalkyle ayant 5 à 11 atomes dans le cycle, bicyclohétéroalcényle ayant 5 à 11 atomes dans le cycle, (C₆-C₁₁)aryle ou hétéroaryle ayant 5 à 12 atomes dans le cycle, et

R₃ et R₄, ou R₄ et R₅, ou R₅ et R₆, peuvent former ensemble, et avec les deux atomes de carbone qui les portent, un autre cycle aromatique ou hétéroaromatique ayant 6 atomes dans le cycle, éventuellement substitué par 1 à 4 substituants choisis parmi ceux définis pour R₇, R₈ et R₉.

Un premier sous-ensemble de composés intéressants est celui des composés de formule générale (I), dans laquelles X représente un groupe de formule générale C-R₂ tel que défini ci-dessus.

Parmi ces composés on peut distinguer, d'une part, les composés de formule générale (I) dans laquelle P, Q, R et W représentent chacun, respectivement, un groupe de formule générale C-R₃, C-R₄, C-R₅ et C-R₆, tels que définis ci-dessus, et, d'autre part, les composés de formule générale (I) dans laquelle l'un des symboles P, Q, R et W représente un atome d'azote.

Un second sous-ensemble de composés intéressants est celui des composés de formule générale (I), dans laquelles X représente un atome d'azote.

Parmi ces composés on peut distinguer, d'une part, les composés de formule générale (I) dans laquelle P, Q, R et W représentent chacun, respectivement, un groupe de formule générale C-R₃, C-R₄, C-R₅ et C-R₆, tels que définis ci-dessus, et, d'autre part, les composés de formule générale (I) dans laquelle l'un des symboles P, Q, R et W représente un atome d'azote.

Les composés de l'invention peuvent exister à l'état de bases ou de sels d'addition à des acides.

Conformément à l'invention, on peut préparer les composés de formule générale (I) par un procédé illustré par le schéma 1 qui suit.

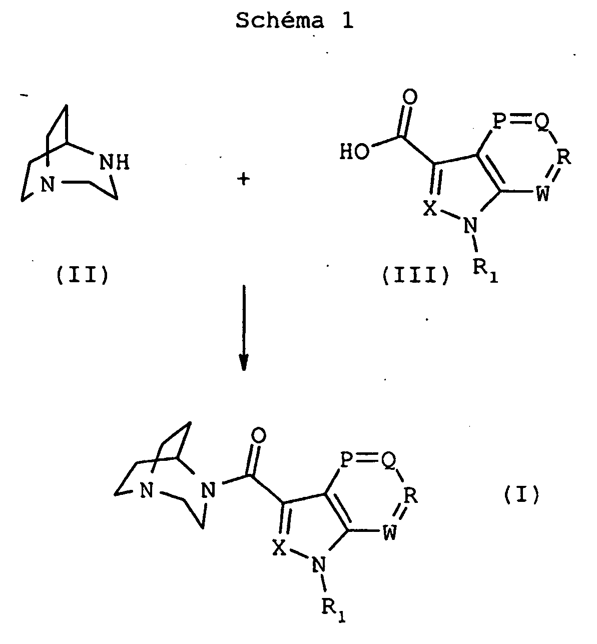

On fait réagir le 1,4-diazabicyclo[3.2.2]nonane de formule (II) avec un composé de formule générale (III), dans laquelle X, P, Q, R, W et R₁ sont tels que définis ci-dessus, en présence d'un agent de couplage tel que, par exemple, le *N,N*'-carbonyldiimidazole, dans un solvant tel que le *N,N*-diméthylformamide. Alternativement, la fonction acide carboxylique présente sur le composé de formule générale (III) peut, dans une étape préalable, être transformée en fonction chlorure d'acide pour réagir avec le 1,4-diazabicyclo[3.2.2]nonane dans un solvant tel que le dichloroéthane.

Les composés de formule générale (I) peuvent aussi être préparés par un procédé illustré par le schéma 2 qui suit.

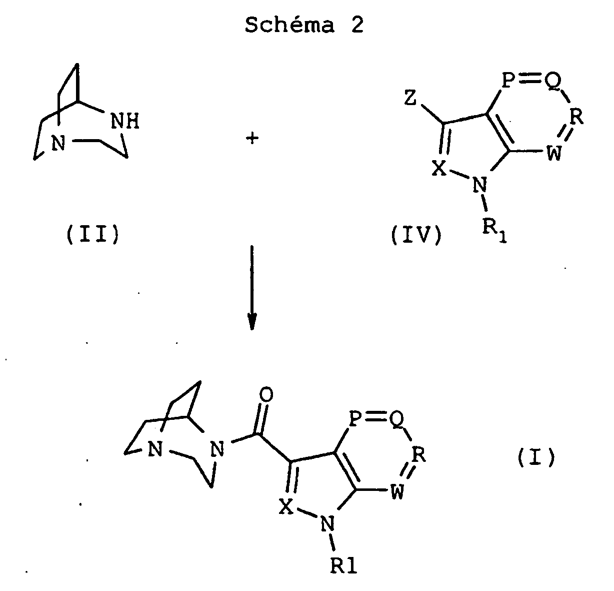

On fait réagir le 1,4-diazabicyclo[3.2.2]nonane de formule (II) avec un composé de formule générale (IV) dans laquelle X, P, Q, R, W et R₁ sont tels que définis ci-dessus, et Z représente un atome de brome ou d'iode, en présence de monoxyde de carbone et d'un catalyseur au palladium tel que, par exemple, le bis(triphénylphosphino)dichloro-palladium, et d'une base telle que, par exemple, la triéthylamine, dans un solvant tel que, par exemple, le *N*,*N*-diméthylformamide.

Pour certains composés, les substituants peuvent ne pas être présents dans le composé de départ de formule générale (III) ou (IV) ; selon leur nature, ces substituants peuvent être introduits sur le composé final de formule générale (I). Ainsi, par exemple des composés de formule générale (I) dans lesquelles P, Q, R et W représentent chacun, respectivement, un groupe de formule générale C-R₃, C-R₄, C-R₅ et C-R₆ où R₃, R₄, R₅ et R₆ représentent chacun un groupement (C₆-C₁₁)aryle ou (C₅-C₁₂)hétéroaryle, peuvent être préparés à partir des composés correspondants, dans la formule desquels R₃, R₄, R₅ et R₆ représentent chacun un atome de brome ou d'iode, selon toutes méthodes connues, telles qu'un couplage de type Suzuki en présence d'un acide boronique et d'un catalyseur au palladium, par exemple le tétrakistriphénylphosphine palladium.

Les composés de formule générale (III) sont accessibles dans le commerce ou par des méthodes décrites dans la littérature, comme par exemple dans Can. J. Chem. 1988, 66, 420-8.

Les composés de formule générale (IV) sont accessibles dans le commerce ou par des méthodes décrites dans la littérature comme, par exemple dans J. Het. Chem. 1983, 475.

La préparation du 1,4-diazabicyclo[3.2.2]nonane est décrite dans J. Med. Chem. 1993, 36, 2311-2320.

Les exemples qui vont suivre illustrent la préparation de quelques composés de l'invention. Les microanalyses élémentaires, et les spectres I.R. et R.M.N. confirment les structures des composés obtenus.

Les numéros indiqués entre parenthèses dans les titres des exemples correspondent à ceux de la 1ère colonne du tableau donné plus loin.

Dans les noms des composés, le tiret "-" fait partie du mot, et le tiret "_" ne sert que pour la coupure en fin de ligne ; il est à supprimer en l'absence de coupure, et ne doit être remplacé ni par un tiret normal ni par un espace.

### Exemple 1 (Composé N°2).

### Chlorhydrate de 3-(1,4-diazabicyclo[3.2.2]non-4-ylcarbonyl)-1H-indazole.

Dans un réacteur de 100 ml on introduit 2,4 g (14,8 mmoles) d'acide 1*H*-indazole-3-carboxylique en solution dans, 30 ml de *N*,*N*-diméthylformamide et 2, g (14,8 mmoles) de *N*,*N*'-carbonyldiimidazole. On agite le mélange à température ambiante pendant 45 min, puis on ajoute 1,7 g (13,4 mmoles) de 1,4-diazabicyclo[3.2.2]nonane en solution dans 20 ml de *N,N*-diméthylformamide, et on agite le mélange à température ambiante pendant 15 h.

On le dilue dans 100 ml d'acétate d'éthyle, on lave la phase organique avec 100 ml d'une solution aqueuse saturée de chlorure de sodium, on la sèche et on la filtre. On concentre le solvant sous pression réduite et on purifie le résidu par chromatographie sur colonne de gel de silice en éluant par un mélange de chloroforme, méthanol et ammoniaque dans les proportions 85/15/1.5. On obtient 1,7 g de produit que l'on dissout dans 30 ml d'alcool isopropylique avant d'ajouter 1,5 ml d'une solution 5N d'acide chlorhydrique dans l'alcool isopropylique. On collecte les cristaux obtenus par filtration et on les sèche sous pression réduite.

On obtient 1,2 g de chlorhydrate.

Point de fusion : 282-283°C.

### Exemple 2 (Composé N°8).

### 3-(1,4-Diazabicyclo[3.2.2]non-4-ylcarbonyl)-1H-pyrazolo[3,4-c]pyridine.

Dans un réacteur de 50 ml on introduit successivement 1 g (5 mmoles) de 3-bromo-1*H*-pyrazolo[3,4-c]pyridine, 0,53 g (0,75 mmoles) de bis(triphénylphosphino)dichloropalladium, 1,9 g (15,1 mmoles) de 1,4-diazabicyclo[3.2.2]nonane et 3,5 ml (25 mmoles) de triéthylamine en solution dans 15 ml de *N,N*-diméthylformamide. On purge ensuite le mélange avec du monoxyde de carbone et on chauffe à 110°C pendant 20 h. On verse le milieu réactionnel dans 100 ml d'eau et on extrait la phase aqueuse par du chloroforme. Après séchage des phases organiques on les sèche, on les filtre et on les concentre sous pression réduite. On purifie le résidu par chromatographie sur colonne de gel de silice en éluant par un mélange de chloroforme, méthanol et ammoniaque dans les proportions 90/10/1.

On obtient ainsi 0,41 g de produit sous forme de solide cristallisé.

Point de fusion : 232-234°C.

### Exemple 3 (Composé N°1).

### Chlorhydrate de 4-(1H-indol-3-ylcarbonyl)-1,4-diazabicyclo_ [3.2.2]nonane.

Par analogie avec l'exemple 1, on fait réagir 0,117 g (0,72 mmole) d'acide 1*H*-indole-3-carboxylique avec 0,083 g (0,66 mmole) de 1,4-diazabicyclo[3.2.2]nonane dans les conditions décrites dans l'exemple 1.

On obtient 0,008 g de chlorhydrate.

Point de fusion : 332-333°C.

### Exemple 4 (Composé N°6).

### Chlorhydrate de 3-(1,4-diazabicyclo[3.2.2]non-4-ylcarbo_ nyl)-1H-pyrrolo[2,3-b]pyridine.

Par analogie avec l'exemple 2 on fait réagir 0,5 g (2,05 mmoles) de 3-iodo-1*H*-pyrrolo[2,3-*b*]pyridine avec 0,52 g (4,1 mmoles) de 1,4-diazabicyclo[3.2.2]nonane dans les conditions décrites dans l'exemple 2.

On obtient 0,13 g de chlorhydrate.

Point de fusion : >300°C.

### Exemple 5 (Composé N°9).

### Bromhydrate de 3-(diazabicyclo[3.2.2]non-4-ylcarbonyl)-6-méthyl-1H-pyrazolo[3,4-b]pyridine.

Par analogie avec l'exemple 2 on fait réagir 0,105 g (0,5 mmole) de 3-bromo-6-méthyl-1*H*-pyrazolo[3,4-*b*]pyridine avec 0,108 g (0,86 mmole) de 1,4-diazabicyclo[3.2.2]nonane dans les conditions décrites dans l'exemple 2.

On obtient 0,16 g de bromhydrate.

Point de fusion : >300°C.

### Exemple 6 (Composé N°27).

### Bromhydrate de 3-(1,4-diazabicyclo[3.2.2]non-4-ylcarbonyl)-5-(5-méthyl-2-thiényl)-1H-indazole.

Dans un réacteur de 10 ml on introduit successivement 0,1 g (0,29 mmole) de 5-bromo-3-(1,4-diazabicyclo[3.2.2]non-4-ylcarbonyl)-1*H*-indazole, 0,057 g (0,4 mmole) d'acide 5-méthyl-2-thiophèneboronique, 0,016 g (0,01 mole) de tétrakis(triphénylphosphino)palladium, 0,058 g (0,27 mmole) de carbonate de sodium, 2 ml de toluène et 0,2 ml d'éthanol et on chauffe le mélange à 100°C pendant 4 h.

On l'hydrolyse par 2 ml d'eau, on extrait la phase aqueuse par du chloroforme, on sèche les phases organiques réunies sur sulfate de magnésium, on les filtre et on les concentre sous pression réduite. On purifie le résidu par chromatographie sur plaque de silice en éluant par un mélange 90/10/1 de chloroforme, méthanol et ammoniaque.

On obtient ainsi 0,083 g de produit que l'on dissout dans 2 ml d'alcool isopropylique pour ajouter 0,08 ml d'une solution 5,7N d'acide bromhydrique dans l'acide acétique. On collecte les cristaux formés par filtration et on les sèche sous vide.

On obtient 0,068 g de produit.

Point de fusion : 339-340°C.

### Exemple 7 (Composé N°83)

### 3-(1,4-Diazabicyclo[3.2.2]non-4-ylcarbonyl)-6-(4-fluoro_ phényl)-1H-pyrrolo[2,3-b]pyridine.

### 7.1. 2,2,2-Trichloro-1-(6-chloro-1H-pyrrolo[2,3-b]pyridin-3-yl)éthanone.

On place dans un ballon avec réfrigérant, agitation magnétique et garde à chlorure de calcium, 4 g (0,03 mole) de trichlorure d'aluminium, 3 ml de dichlorométhane, 3,6 g (0,02 mole), soit 2,23 ml, de chlorure de trichloroacétyle et on agite le mélange à température ambiante pendant 30 min.

On ajoute, par petites fractions, 1,52 g (0,01 mole) de 6-chloro-1*H*-pyrrolo[2,3-*b*]pyridine et on chauffe le mélange au reflux pendant 3 h.

En le refroidissant dans un bain de glace, on ajoute 50 ml de glace dans le ballon et on agite vigoureusement pendant 30 min.

On obtient une masse pâteuse qu'on sépare de la phase aqueuse par décantation.

On utilise le produit directement dans l'étape suivante.

### 7.2. Acide 6-chloro-1H-pyrrolo[2,3-b]pyridine-3-carbo_ xylique.

On reprend le dérivé brut de l'étape précédente par 10 ml d'eau et 6 ml de lessive de soude à 35% et on chauffe le mélange dans un bain d'huile à 90°C pendant 10 min.

Après refroidissement on filtre le milieu réactionnel, on refroidit le filtrat dans un bain de glace et on l'acidifie à pH environ 3 avec de l'acide chlorhydrique concentré.

On obtient une précipitation blanche qu'on filtre, essore et sèche au dessiccateur sous vide.

On isole 1,72 g de produit.

Point de fusion : 283-284°C.

### 7.3. 6-Chloro-3-(1,4-diazabicyclo[3.2.2]non-4-ylcarbonyl)-1H-pyrrolo[2,3-b]pyridine (Composé N°82).

Dans un tube à micro ondes (Personal Chemistry) on place 375 mg (2 mmoles) du dérivé de l'étape précédente, 760 mg (4 mmoles) de chlorhydrate de 1-(3-diméthylaminopropyl)-3-éthylcarbodiimide, 50 mg de diméthylaminopyridine, 5 ml d'acétonitrile et 500 mg (4 mmoles) de diazabicyclononane, on capsule le tube et on le chauffe à 100°C pendant 10 min. On noie le mélange dans 50 ml d'eau, on l'extrait au dichlorométhane et on évapore la phase organique.

Par trituration dans l'éther isopropylique on obtient 310 mg d'un composé cristallisé.

Point de fusion : 228-229°C.

### 7.4. 3-(1,4-Diazabicyclo[3.2.2]non-4-ylcarbonyl)-

6-(4-fluorophényl)-1*H*-pyrrolo[2,3-*b*]pyridine. Dans un tube à micro ondes (Personal Chemistry) on place 87 mg (0,287 mmole) du dérivé obtenu à l'étape précédente, 80 mg (2 équivalents) d'acide (4-fluorophényl)boronique, 20 mg de tétrakis(triphénylphosphine)palladium(0), 2 ml de toluène, 2 ml d'acétonitrile, 2 ml de solution 2N de carbonate de sodium et on chauffe le mélange à 150°C pendant 10 min.

On dépose la phase organique sur une cartouche de silice et on élue avec un mélange 90/10/1 de dichlorométhane, méthanol et ammoniaque.

Le résidu huileux obtenu cristallise par trituration dans l'éther isopropylique.

On en isole 60 mg.

Point de fusion : 285-286°C.

Le tableau qui suit illustre les structures chimiques et les propriétés physiques de quelques composés de l'invention.

Dans les colonnes P, Q, R et W, "Ms" désigne un groupe méthylsulfonyle, iC₃H₇ désigne un groupe 1-méthyléthyle, x-C₄H₃S désigne un groupe x-thiényle, x-C₄H₃O désigne un groupe x-furyle, x-C₄H₄N désigne un groupe x-pyrrolyle, x-C₅H₄N désigne un groupe x-pyridinyle.

Dans la colonne "Sel", "-" désigne un composé à l'état de base, "HBr" désigne un bromhydrate, "HCl" désigne un chlorhydrate et "ox." désigne un oxalate.

**Tableau**

| | | | | | | | | |
|---|---|---|---|---|---|---|---|---|
| 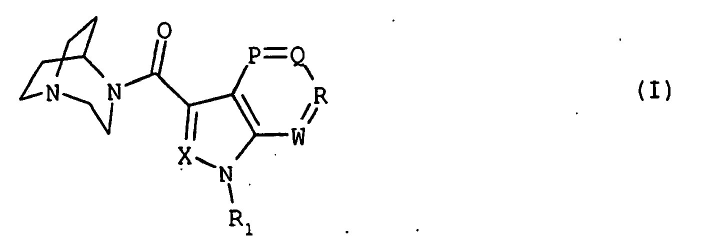 | | | | | | | | |

| N° | X | R₁ | P | Q | R | W | Sel | F (°C) |
|---|---|---|---|---|---|---|---|---|
| 1 | CH | H | CH | CH | CH | CH | HCl | 332-333 |
| 2 | N | H | CH | CH | CH | CH | HCl | 282-283 |
| 3 | CH | CH₃ | CH | CH | CH | CH | HCl | 227-228 |
| 4 | N | H | CH | CH | C-CH₃ | CH | HCl | 339-340 |
| 5 | N | H | CH | CH | C-NO₂ | CH | - | 209-210 |
| 6 | CH | H | CH | CH | CH | N | HCl | >300 |
| 7 | N | H | CH | CH | CH | N | HCl | >325 |
| 8 | N | H | CH | CH | N | CH | - | 232-234 |
| 9 | N | H | CH | CH | C-CH₃ | N | HBr | >300 |
| 10 | N | H | CH | C-F | CH | CH | HBr | 337-338 |
| 11 | N | H | CH | CH | C-Cl | CH | HBr | 336-337 |
| 12 | N | H | CH | C-Br | CH | CH | HBr | 364-365 |
| 13 | N | H | Ch | C-OMs | CH | CH | ox. | 242-243 |
| 14 | Ch | H | Ch | C-C₆H₅ | CH | CH | HCl | 322-323 |
| 15 | Ch | H | Ch | C-(4-F-C₆H₄) | CH | CH | HCl | 356-357 |
| 16 | CH | H | CH | C-(4-Cl-C₆H₄) | CH | CH | HCl | 316 |
| 17 | CH | H | CH | C-(4-CH₃-C₆H₄) | CH | CH | HCl | 289-290 |
| 18 | CH | H | CH | C-(4-(CH₂)₄-C₆H₄) | CH | CH | HCl | 350-351 |
| 19 | CH | H | CH | C-(4-CF₃-C₆H₄) | CH | CH | HCl | 293-294 |
| 20 | CH | H | CH | C-(4-OCH₃-C₆H₄) | CH | CH | HCl | 300-301 |
| 21 | CH | H | CH | C-(4-OCF₃-C₆H₄) | CH | CH | HCl | 292 |
| 22 | CH | H | CH | C-(3-Cl-4-F-C₆H₃) | CH | CH | HCl | 321-322 |
| 23 | CH | H | CH | C-(3,4-Cl₂-C₆H₃) | CH | CH | HCl | |
| 24 | CH | H | CH | C-(2,3-Cl₂-C₆H₃) | CH | CH | HCl | 260-261 |
| 25 | CH | H | CH | C-(3-F-4-OCH₃-C₆H₅) | CH | CH | HCl | 287-288 |
| 26 | N | H | CH | C-CH₃ | CH | CH | HBr | 291-292 |
| 27 | N | H | CH | C-(5-CH₃-2-C₄H₃S) | CH | CH | HBr | 339-340 |
| 28 | N | H | CH | C-Cl | CH | CH | - | 248-249 |
| 29 | CH | H | CH | C-(3-F-C₆H₄) | CH | CH | HCl | 250-252 |
| 30 | CH | H | CH | C-(3-CN-C₆H₄) | CH | CH | HCl | 221-223 |
| 31 | CH | H | CH | C-(3-CH₃-C₆H₄) | CH | CH | HCl | 367-369 |
| 32 | CH | H | CH | C-(3-(CH(CH₃)₂-C₆H₄) | CH | CH | HCl | 174-176 |
| 33 | CH | H | CH | C-(3-CF₃-C₆H₄) | CH | CH | ox. | 258-260 |
| 34 | CH | H | CH | C-(3-OCH₃-C₆H₄) | CH | CH | HCl | 199-201 |
| 35 | CH | H | CH | C-(3-OCH₂CH₂CH₃-C₆H₄) | CH | CH | HCl | 263-265 |
| 36 | CH | H | CH | C-3-C₄H₃S | CH | CH | HCl | 279-281 |
| 37 | CH | H | CH | C-2-C₄H₃S | CH | CH | HCl | 231-233 |
| 38 | CH | H | CH | C-(5-CH₃-2-C₄H₃S) | CH | CH | HCl | 280-282 |
| 39 | CH | H | CH | C-(4-CH₃-2-C₄H₃S) | CH | CH | HCl | 369-371 |
| 40 | CH | H | CH | C-2-C₄H₃O | CH | CH | HCl | 258-260 |
| 41 | CH | H | CH | C-3-C₄H₃O | CH | CH | HCl | 313-315 |
| 42 | N | H | CH | C-OCH₃ | CH | CH | - | 212-213 |
| 43 | N | H | CH | CH | C-CF₃ | CH | HBr | 290-292 |
| 44 | N | H | CH | CH | C-Br | CH | - | 195-196 |
| 45 | N | H | CH | CH | C-2-C₄H₃S | CH | - | 263-264 |
| 46 | N | H | CH | C-2-C₅H₃S | CH | CH | HBr | 329-330 |
| 47 | N | H | N | CH | CH | CH | HBr | 312-315 |
| 48 | N | H | CH | CH | C-2-C₄H₃O | CH | - | 242-243 |
| 49 | N | H | CH | CH | C-NH₂ | CH | ox. | 295-296 |
| 50 | N | H | CH | C-2-C₄H₃O | CH | CH | HBr | 321-322 |
| 51 | CH | H | CH | CH | CH | C-C₆H₅ | HCl | 222-224 |
| 52 | CH | H | CH | CH | CH | C-(4-F-C₆H₄) | HCl | 264-266 |
| 53 | CH | H | CH | CH | CH | C-(4-Cl-C₆H₄) | HCl | 369-371 |
| 54 | CH | H | CH | CH | CH | C-(4-CH₃-C₆H₄) | HCl | 303-305 |
| 55 | CH | H | CH | CH | CH | C-(4-C₄H₉-C₆H₄) | HCl | 254-256 |
| 56 | CH | H | CH | CH | CH | C-(4-CF₃-C₆H₄) | HCl | 226-228 |
| 57 | CH | H | CH | CH | CH | C-(4-OCH₃-C₆H₄) | HCl | 306-308 |
| 58 | CH | H | CH | CH | CH | C-(4-OCF₃-C₆H₄) | HCl | 305-307 |
| 59 | CH | H | CH | CH | CH | C-(3-F-C₆H₄) | HCl | 286-288 |
| 60 | CH | H | CH | CH | CH | C-(3-CN-C₆H₄) | HCl | 189-191 |
| 61 | CH | H | CH | CH | CH | C-(3-CH₃-C₆H₄) | HCl | 261-263 |
| 62 | CH | H | CH | CH | CH | C-(4-iC₃H₇-C₆H₄) | HCl | 293-295 |
| 63 | CH | H | CH | CH | CH | C-(3-OCF₃-C₆H₄) | HCl | 302-304 |
| 64 | CH | H | CH | CH | CH | C-3-C₄H₃S | HCl | 317-319 |
| 65 | CH | H | CH | CH | CH | C-(5-CH₃-2-C₄H₃S) | HCl | |
| 66 | CH | H | CH | CH | CH | C-3-C₄H₃O | HCl | |
| 67 | CH | H | CH | CH | CH | C-4-C₅H₄N | HCl | |
| 68 | CH | H | CH | CH | CH | C-Br | - | 197-199 |
| 69 | CH | H | CH | CH | CH | C-3-C₅H₄N | HCl | |
| 70 | N | H | CH | C-2-C₄H₄N | CH | CH | ox. | 171-172 |
| 71 | CH | H | CH | C-Br | CH | CH | - | 237-239 |
| 72 | CH | H | CH | CH | C-Br | CH | - | 237-239 |
| 73 | N | H | C-Cl | CH | CH | CH | HCl | 339-340 |
| 74 | N | H | CH | CH | CH | C-CH₃ | HCl | 327-328 |
| 75 | N | H | CH | CH | CH | C-Cl | HCl | 316-317 |
| 76 | N | H | CH | N | CH | CH | - | 217-219 |
| 77 | N | H | CH | CH | CH | C-OCH₃ | - | 272-273 |
| 78 | N | H | CH | C-CH₃ | CH | C-Cl | HCl | 313-314 |
| 79 | N | H | C-F | CH | CH | CH | HBr | 302-303 |
| 80 | N | H | CH | C-Br | CH | C-CH₃ | HBr | 305-306 |
| 81 | N | H | CH | CH | C-3-C₄H₃O | CH | HBr | 331-332 |
| 82 | CH | H | CH | CH | C-Cl | N | - | 228-229 |
| 83 | CH | H | CH | CH | C-(4-F-C₆H₄) | N | - | 285-286 |
| 84 | N | H | CH | C-NH₂ | CH | CH | ox. | 144-145 |
| 85 | N | H | CH | C-4-C₅H₄N | CH | CH | ox. | 281-282 |
| 86 | N | H | CH | C-3-C₄H₃O | CH | CH | ox. | 310-311 |
| 87 | N | H | CH | CH | 2-C₄H₄N | CH | HBr | 324-325 |

Les composés de la présente invention ont été étudiés quant à leur affinité vis à vis des récepteurs nicotiniques contenant la sous-unité α₄β₂ selon les méthodes décrites par Anderson et Arneric dans Eur. J. Pharmacol. 1994, 253, 261 et par Hall et coll. dans Brain Res. 1993, 600, 127.

On décapite des rats mâles Sprague Dawley de 150 à 200 g et on prélève rapidement la totalité du cerveau, on l'homogénéise dans 15 volumes d'une solution de sucrose à 0,32 M à 4°C puis on le centrifuge à 1000 G pendant 10 min. On élimine le culot et centrifuge le surnageant à 20000 G pendant 20 min à 4°C. On récupère le culot et on l'homogénéise à l'aide d'un broyeur Polytron™ dans 15 volumes d'eau bidistillée à 4°C, puis on le centrifuge à 8000 G pendant 20 min. On élimine le culot et on centrifuge le surnageant et la couche de peau (buffy coat) à 40000 G pendant 20 min, on récupère le culot, on le remet en suspension dans 15 ml d'eau bidistillée et on le centrifuge encore une fois à 40000 G avant de le conserver à -80°C.

Le jour de l'expérience on décongèle lentement le tissu et on le met en suspension dans 3 volumes de tampon. On fait incuber 150 µl de cette suspension membranaire à 4°C pendant 120 min. En présence de 100 µl de [³H]-cytisine à 1 nM dans un volume final de 500 µl de tampon, en présence ou en absence de composé à tester. On arrête la réaction par filtration sur des filtres Whatman GF/B™ préalablement traités avec de la polyéthylènimine, on rince les filtres avec deux fois 5 ml de tampon à 4°C, et on mesure la radioactivité retenue sur le filtre par scintigraphie liquide. On détermine la liaison non spécifique en présence de (-)-nicotine à 10 µM ; la liaison non spécifique représente 75 à 85% de la liaison totale récupérée sur le filtre. Pour chaque concentration de composé étudié on détermine le pourcentage d'inhibition de la liaison spécifique de [³H]-cytisine, puis on calcule la CI₅₀, concentration de composé qui inhibe 50% de la liaison spécifique.

Les CI₅₀ des composés de l'invention les plus affins se situent entre 1 et 10 µM.

Les composés de l'invention ont aussi été étudiés quant à leur affinité vis à vis des récepteurs nicotiniques contenant la sous unité α₇, selon les méthodes décrites par Mark et Collins dans J. Pharmacol. Exp. Ther. 1982, 22, 564 et par Marks et coll. dans Mol. Pharmacol. 1986, 30, 427.

On décapite des rats mâles OFA de 150 à 200 g, on prélève rapidement la totalité du cerveau, on l'homogénéise à l'aide d'un broyeur Polytron™ dans 15 volumes d'une solution de sucrose à 0,32 M à 4 °C, puis on le centrifuge à 1000 G pendant 10 min. On élimine le culot et on centrifuge le surnageant à 8000 G pendant 20 min à 4 °C. On récupère le culot et on l'homogénéise à l'aide d'un broyeur Polytron™ dans 15 volumes d'eau bidistillée à 4°C, puis on le centrifuge à 8000 G pendant 20 min. On élimine le culot et on centrifuge le surnageant et la couche de peau ("buffy coat") à 40000 G pendant 20 min. On récupère le culot, on le remet en suspension avec 15 volumes d'eau bidistillée à 4°C et on le centrifuge encore une fois à 40000 G pendant 20 min avant de le conserver à -80°C.

Le jour de l'expérience on décongèle lentement le tissu et on le met en suspension dans 5 volumes de tampon. On préincube 150 µl de cette suspension membranaire à 37°C pendant 30 min, à l'obscurité, en présence ou en absence du composé à tester. Puis les membranes sont incubées pendant 60 min à 37°C, à l'obscurité, en présence de 50 µl de [³H]α-bungarotoxine à 1 nM dans un volume final de 250 µl de tampon HEPES 20 mM, polyéthylènimine 0,05%. On arrête la réaction par filtration sur des filtres Whatman GF/C™ préalablement traités pendant 3 h avec de la polyéthylènimine à 0,05%. On rince les filtres avec deux fois 5 ml de tampon à 4°C et on mesure la radioactivité retenue sur chaque filtre par scintigraphie liquide. On détermine la liaison non spécifique en présence de α-bungarotoxine à 1 µM finale ; la liaison non spécifique représente environ 60 % de la liaison totale récupérée sur le filtre. Pour chaque concentration de composé étudié on détermine le pourcentage d'inhibition de la liaison spécifique de [³H]α-bungarotoxine, puis on calcule la CI₅₀, concentration de composé qui inhibe 50% de la liaison spécifique.

Les CI₅₀ des composés de l'invention les plus affins se situent entre 0,005 et 0,15 µM.

Les résultats qui précèdent montrent que les composés de l'invention sont des ligands sélectifs pour les sous unités α₇ du récepteur nicotinique.

Les résultats des divers essais suggèrent l'utilisation des composés dans le traitement ou la prévention des désordres liés à un dysfonctionnement des récepteurs nicotiniques, notamment au niveau du système nerveux central.

Ces désordres comprennent les altérations cognitives, plus spécifiquement mnésiques, mais également attentionnelles, liées à la maladie d'Alzheimer, au vieillissement pathologique (Age Associated Memory Impairment, AAMI), au syndrome Parkinsonien, à la trisomie 21 (Down's syndrome), au syndrome alcoolique de Korsakoff, aux démences vasculaires (multi-infarct dementia, MDI).

Les composés de l'invention pourraient également être utiles dans le traitement des troubles moteurs observés dans la maladie de Parkinson ou d'autres maladies neurologiques telles que la chorée de Huntington, le syndrome de Tourette, la dyskinésie tardive et l'hyperkinésie.

Les composés de l'invention peuvent également constituer un traitement curatif ou symptomatique des pathologies neurodégénératives aiguës telles que les accidents vasculaires cérébraux et des épisodes hypoxiques cérébraux, ainsi que des pathologies neurodégénératives chroniques comme la maladie d'Alzheimer. Ils peuvent aussi être utilisés dans les cas de pathologies psychiatriques : schizophrénie, dépression, anxiété, attaques de panique, comportements compulsifs et obsessionnels.

Ils peuvent prévenir les symptomes dus au sevrage au tabac, à l'alcool, aux différentes substances induisant une dépendance, telles que cocaïne, LSD, cannabis, benzodiazepines.

On sait, par ailleurs, que l'activation des récepteurs nicotiniques vasculaires stimule la prolifération des cellules endothéliales et musculaires lisses artérielles, réduit leur apoptose, et accroît la formation des tubes capillaires in vitro. In vivo, ces effets se traduisent par une action angiogénique bien documentée dans des modèles d'ischémie des membres inférieurs. La nicotine administrée chroniquement augmente la densité capillaire, le diamètre des artères collatérales, et la perfusion sanguine dans le muscle squelettique ischémié. Les effets angiogéniques et artériogéniques de la nicotine apparaissent concomitamment à l'augmentation du recrutement des monocytes, et sont médiés en partie par la libération de facteurs angiogéniques comme le monoxyde d'azote, et le VEGF (vascular endothelial growth factor). Par son activité angiogénique, la nicotine, appliquée en topique, accélère la cicatrisation cutanée chez les animaux diabétiques.

Compte-tenu des activités résumées ci-dessus, les indications thérapeutiques sont le traitement de l'ischémie des membres inférieurs, de l'artérite oblitérante des membres inférieurs (PAD : peripheral arterial disease), de l'ischémie cardiaque (angor stable), de l'infarctus du myocarde, de l'insuffisance cardiaque, du déficit de cicatrisation cutanée des patients diabétiques, des ulcères variqueux de l'insuffisance veineuse.

Pour chacune des pathologies pré-citées, le traitement se fera avec l'agent nicotinique seul et/ou en association avec les médicaments de référence indiqués dans la pathologie. »

C'est pourquoi la présente invention a également pour objet des compositions pharmaceutiques contenant une dose efficace d'au moins un composé selon l'invention, à l'état de base ou de sel ou de solvat pharmaceutiquement acceptable, et en mélange, le cas échéant, avec des excipients convenables.

Lesdits excipients sont choisis selon la forme pharmaceutique et le mode d'administration souhaité.

Les compositions pharmaceutiques selon l'invention peuvent ainsi être destinées à l'administration orale, sublinguale, sous-cutanée, intramusculaire, intraveineuse, topique, intratrachéale, intranasale, transdermique, rectale, intraocculaire.

Les formes unitaires d'administration peuvent être, par exemple, des comprimés, des gélules, des granules, des poudres, des solutions ou suspensions orales ou injectables, des timbres transdermiques ("patch"), des suppositoires. Pour l'administration topique on peut envisager des pommades, lotions et collyres.

Lesdites formes unitaires sont dosées pour permettre une administration journalière de 0,01 à 20 mg de principe actif par kg de poids corporel, selon la forme galénique.

Pour préparer des comprimés on ajoute au principe actif, micronisé ou non, un véhicule pharmaceutique qui peut être composé de diluants, comme par exemple le lactose, la cellulose microcristalline, l'amidon, et des adjuvants de formulation comme des liants, (polyvinylpyrrolidone, hydroxypropylméthylcellulose, etc), des agents d'écoulement comme la silice, des lubrifiants comme le stéarate de magnésium, l'acide stéarique, le tribehenate de glycerol, le stéarylfumarate de sodium. Des agents mouillants ou tensioactifs tels que le laurylsulfate de sodium peuvent aussi être ajoutés.

Les techniques de réalisation peuvent être la compression directe, la granulation sèche, la granulation humide ou la fusion à chaud.

Les comprimés peuvent être nus, dragéifiés, par exemple par du saccharose, ou enrobés avec divers polymères ou autres matières appropriées. I1 peuvent être conçus pour permettre une libération rapide, retardée ou prolongée du principe actif grâce à des matrices polymères ou à des polymères spécifiques utilisés dans l'enrobage.

Pour préparer des gélules on mélange le principe actif avec des véhicules pharmaceutiques secs (simple mélange, granulation sèche ou humide, ou fusion à chaud), liquides ou semi-solides.

Les gélules peuvent être dures ou molles, pelliculées ou non, de manière à avoir une activité rapide, prolongée ou retardée (par exemple pour une forme entérique).

Une composition sous forme de sirop ou d'élixir ou pour l'administration sous forme de gouttes peut contenir le principe actif conjointement à un édulcorant, de préférence acalorique, du méthylparaben ou du propylparaben comme antiseptique, un agent de sapidité et un colorant.

Les poudres et granules dispersibles dans de l'eau peuvent contenir le principe actif en mélange avec des agents de dispersion ou des agents mouillants, ou des agents dispersants comme la polyvinylpyrrolidone, de mêmes qu'avec des édulcorants et des agents correcteurs de goût.

Pour l'administration rectale, on recourt à des suppositoires préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles injectables contenant des agents de dispersion et/ou des mouillants pharmacologiquement compatibles, par exemple le propylèneglycol ou le butylène-glycol.

Le principe actif peut être formulé également sous forme de microcapsules, éventuellement avec un ou plusieurs supports ou additifs, ou bien avec une matrice polymère ou avec une cyclodextrine (timbres transdermiques, formes à libération prolongée).

Les compositions topiques selon l'invention comprennent un milieu compatible avec la peau. Elles peuvent se présenter notamment sous forme de solutions aqueuses, alcooliques ou hydroalcooliques, de gels, d'émulsions eau-dans-huile ou huile-dans-eau ayant l'aspect d'une crème ou d'un gel, de microémulsions, d'aérosols, ou encore sous forme de dispersions vésiculaires contenant des lipides ioniques et/ou non ioniques. Ces formes galéniques sont préparées selon les méthodes usuelles des domaines considérés.

Enfin, les compositions pharmaceutiques selon l'invention peuvent contenir, à côté d'un composé de formule générale (I), d'autres principes actifs qui peuvent être utiles dans le traitement des troubles et maladies indiqués ci-dessus.

## Revendications

1. Composé de formule générale (I) 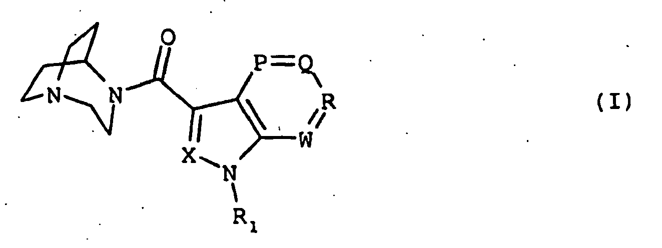 dans laquelle
X représente un atome d'azote ou un groupe de formule générale C-R₂;
P représente un groupe de formule générale C-R₃;
Q représente un groupe de formule générale C-R₄;
R représente un groupe de formule générale C-R₅;
W représente un groupe de Formule générale C-R₆;
ou bien l'un des symboles P, Q, R et W représente un atome d'azote,
R₁ représente un atome d'hydrogène ou un groupe (C₁-C₆)alkyle,
R₂ représente un atome d'hydrogène ou un groupe (C₁-C₆)alkyle,
R₃, R₄, R₅ et R₆ représentent chacun, indépendamment l'un de l'autre, un atome d'hydrogène ou d'halogène, ou un groupe (C₁-C₆)alkyle, (C₁-C₆)alcoxy, nitro, amino, trifluorométhyle, cyano, ou de formule générale -NR₇R₈, -NR₇C(=O)R₈, -NR₇C(=O)NR₈R₉, -NR₇C(=O)OR₈, -NR₇S(=O)₂NR₈R₉, -OR₇, -OC(=O)R₇, -OC(=O)OR₇, -OC(=O)ONR₇R₈, -OC(=O)SR₇, -OS(=O)₂R₇, -C(=O)OR₇, - C(=O)R₇, -C(=O)NR₇R₈, -SR₇, -S(=O)R₇, -S(=O)₂R₇, -S(=O)₂NR₇R₈, (C₆-C₁₁)aryle ou (C₃-C₁₂)hétéroaryle éventuellement substitué par un ou plusieurs groupes choisis parmi les atomes d'halogènes, les groupes (C₁-C₆)alkyles, (C₁-C₆)alcoxy, nitro, amino, trifluorométhyles, cyano, ou de formule générales -NR₇R₈ ou -OR₇,
R₇, R₈, et R₉ représentent chacun, indépendamment l'un de l'autre un atome d'hydrogène ou un groupe (C₁-C₆)alkyle linéaire ou branché, un groupe (C₂-C₆)alcényle linéaire ou ramifié, ou un groupe (C₂-C₆)alcynyle linéaire ou ramifié ou un groupe (C₃-C₈)cycloalkyle, (C₄-C₈) cycloalcényle, ou un groupe hétérocycloalkyle ayant 3 à 8 atomes dans le cycle ou un groupe (C₅-C₁₁)bicycloalkyle, (C₇-C₁₁)bicycloalcényle, hétérobicycloalkyle ayant 5 à 11 atomes dans le cycle, bicyclohétéroalcényle ayant 5 à 11 atomes dans le cycle, (C₆-C₁₁)aryle ou hétéroaryle ayant 5 à 12 atomes dans le cycle,
R₇, R₈ et R₉ peuvent être éventuellement substitués, indépendamment l'un de l'autre, par un atome d'halogène, ou un groupe (C₁-C₆)alkyle, (C₁-C₆)alcoxy, nitro, amino, trifluorométhyle, cyano, ou de formule générale -NR₁₀R₁₁, -NR₁₀C(=O)R₁₁, -NR₁₀C(=O)NR₁₁R₁₂, -NR₁₀C(=O)OR₁₁, -NR₁₀S(=O)₂NR₁₁R₁₂, -OR₁₀, -OC(=O)R₁₀, -OC(=O)OR₁₀, -OC(=O)ONR₁₀R₁₁, -OC(=O)SR₁₀, -C(=O)OR₁₀, -C(=O)R₁₀, -C(=O)NR₁₀R₁₁, -SR₁₀, -S(=O)R₁₀, -S(=O)₂R₁₀, -S(=O)₂NR₁₀R₁₁,
R₁₀, R₁₁ et R₁₂ représentent chacun, indépendamment l'un de l'autre, un atome d'hydrogène ou un groupe (C₁-C₆)alkyle linéaire ou ramifié, un groupe (C₂₋C₆)alcényle linéaire ou ramifié, ou un groupe (C₂-C₆)alcynyle linéaire ou ramifié ou un groupe (C₃-C₈)cycloalkyle, (C₄-C₈)cycloalcényle, ou un groupe hétérocycloalkyle ayant 3 à8 atomes dans le cycle ou un groupe (C₅-C₁₁)bicycloalkyle, (C₇-C₁₁)bicycloalcényle, hétérobicycloalkyle ayant 5 à 11 atomes dans le cycle, bicyclohétéroalcényle ayant 5 à 11 atomes dans le cycle, (C₆-C₁₁)aryle ou hétéroaryle ayant 5 à 12 atomes dans le cycle,
R₁₀, R₁₁ et R₁₂ peuvent être éventuellement substitués, indépendamment l'un de l'autre, par un atome d'halogène, ou un groupe (C₁-C₆)alkyle, (C₁-C₆)alcoxy, nitro, amino, trifluorométhyle, cyano, ou -NR₁₃R₁₄, -NR₁₃C(=O)R₁₄, -NR₁₃C(=O)NR₁₄R₁₅, -NR₁₃C(=O)OR₁₄, -NR₁₃S(=O)₂NR₁₄R₁₅, -OR₁₃, -OC(=O)R₁₃, -OC(=O)OR₁₃, -OC(=O)ONR₁₃R₁₄, -OC(=O)SR₁₃, -C(=O)OR₁₃, -C(=O)R₁₃, -C(=O)NR₁₃R₁₄, -SR₁₃, -S(=O)R₁₃, -S(=O)₂R₁₃, -S(=O)₂NR₁₃R₁₄,
R₁₃, R₁₄ et R₁₅ représentent chacun, indépendamment l'un de l'autre, un atome d'hydrogène ou un groupe (C₁-C₆)alkyle linéaire ou ramifié, un groupe (C₂-C₆)alcényle linéaire ou ramifié, ou un groupe (C₂-C₆)alcynyle linéaire ou ramifié, ou un groupe (C₃-C₈)cycloalkyle, (C₄-C₈)cycloalcényle, ou un groupe hétérocycloalkyle ayant 3 à 8 atomes dans le cycle, ou un groupe (C₅-C₁₁)bicycloalkyle, (C₇-C₁₁)bicycloalcényle, hétérobicycloalkyle ayant 5 à 11 atomes dans le cycle, bicyclohétéroalcényle ayant 5 à 11 atomes dans le cycle, (C₆-C₁₁)aryle ou hétéroaryle ayant 5 à 12 atomes dans le cycle, et
R₃ et R₄, ou R₄ et R₅, ou R₅ et R₆, peuvent former ensemble, et avec les deux atomes de carbone qui les portent, un autre cycle aromatique ou hétéroaromatique ayant 6 atomes dans le cycle, éventuellement substitué par 1 à 4 substituants choisis parmi ceux définis pour R₇, R₈ et R₉, à l'état de base ou de sel d'addition à un acide.

2. Composé selon la revendication 1, **caractérisé en ce que** X représente un groupe de formule générale C-R₂.

3. Composé selon la revendication 2, **caractérisé en ce que** P, Q, R et W représentent chacun, respectivement, un groupe de formole générale C-R₃, C-R₄, C-R₅ et C-R₆.

4. Composé selon la revendication 2, **caractérisé en ce que** l'un des symboles P, Q, R et W représente un atome d'azote.

5. Composé selon la revendication 1**, caractérisé en ce que** X représente un atome d'azote.

6. Composé selon la revendication 5, **caractérisé en ce que** P, Q, R et W représentent, chacun, respectivement, un groupe de formule générale C-R₃, C-R₄, C-R₅ et C-R₆.

7. Composé selon la revendication 5, **caractérisé en ce que** l'un des symboles P, Q, R et W représente un atome d'azote.

8. Procédé de préparation d'un composé selon la revendication 1, **caractérisé en ce qu'**on fait réagir le 1,4-diazabicyclo[3.2.2]nonane avec un composé de formule générale (III) 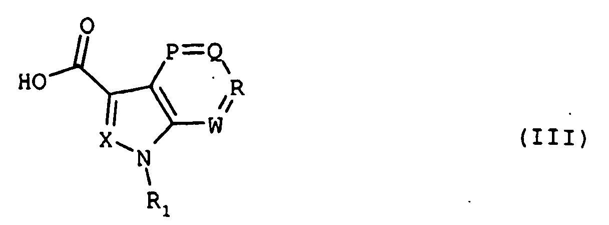 dans laquelle X, P, Q, R, W et R₁ sont tels que définis dans la revendication 1, en présence d'un agent de couplage et dans un solvant, éventuellement après une étape préalable de transformation de la fonction acide carboxylique en fonction chlorure d'acide.

9. Procédé de préparation d'un composé selon la revendication 1, **caractérisé en ce qu'**on fait réagir le 1,4-diazabicyclo[3.2.2]nonane avec un composé de formule générale (IV) 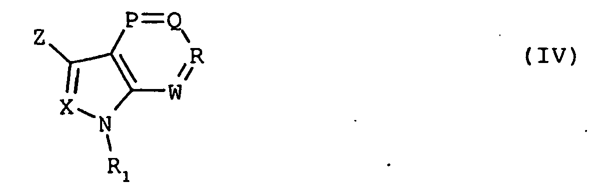 dans laquelle X, P, Q, R, W et R₁ sont tels que définis dans la revendication 1 et Z représente un atome de brome ou d'iode, en présence de monoxyde de carbone, d'un catalyseur au palladium et d'une base, dans un solvant.

10. Composition pharmaceutique, **caractérisée en ce qu'**elle contient un composé selon la revendication 1 et un excipient.

## Claims

1. Compound of general formula (I) 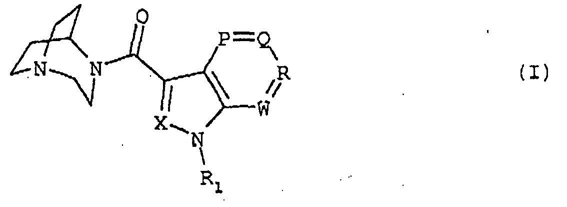 in which
X represents a nitrogen atom or a group of general formula C-R₂,
P represents a group of general formula C-R₃,
Q represents a group of general formula C-R₄,
R represents a group of general formula C-R₅,
W represents a group of general formula C-R₆,
or one of the symbols P, Q, R and W represents a nitrogen atom,
R₁ represents a hydrogen atom or a (C₁-C₆)alkyl group, R₂ represents a hydrogen atom or a (C₁-C₆)alkyl group, R₃, R₄, R₅ and R₆ each represent, independently of each other, a hydrogen or halogen atom, or a (C₁-C₆)alkyl, (C₁-C₆)alkoxy, nitro, amino, trifluoromethyl or cyano group, or a group of general formula -NR₇R₈, -NR₇C(=O)R₈, -NR₇C(=O)NR₈R₉, -NR₇C (=O) OR₈, -NR₇S(=O)₂NR₈R₉, -OR₇, -OC(=O)R₇, -OC(=O)OR₇, -OC(=O)ONR₇R₈, -OC(=O)SR₇, -OS(=O)₂R₇, -C(=O)OR₇, -C(=O)R₇, -C(=O)NR₇R₈, -SR₇, -S(=O)R₇, -S(=O)₂R₇, -S(=O)₂NR₇R₈, or a (C₆-C₁₁)aryl or (C₃-C₁₂)heteroaryl group optionally substituted with one or more groups chosen from halogen atoms, (C₁-C₆)alkyl, (C₁-C₆)alkoxy, nitro, amino, trifluoromethyl or cyano groups, or groups of general formula -NR₇R₈ or -OR₇, R₇, R₈ and R₉ each represent, independently of each other, a hydrogen atom or a linear or branched (C₁-C₆)-alkyl group, a linear or branched (C₂-C₆)alkenyl group, a linear or branched (C₂-C₆)alkynyl group, a (C₃-C₈)-cycloalkyl or (C₄-C₈)cycloalkenyl group, or a hetero-cycloalkyl group containing 3 to 8 atoms in the ring, or a (C₅-C₁₁)bicycloalkyl or (C₇-C₁₁)bicycloalkenyl group, a heterobicycloalkyl group containing 5 to 11 atoms in the ring, a bicycloheteroalkenyl group containing 5 to 11 atoms in the ring, or a (C₆-C₁₁)aryl or heteroaryl group containing 5 to 12 atoms in the ring,
R₇, R₈ and R₉ may be optionally substituted, independently of each other, with a halogen atom or a (C₁-C₆)alkyl, (C₁-C₆)alkoxy, nitro, amino, trifluoromethyl or cyano group, or a group of general formula -NR₁₀R₁₁, -NR₁₀C(=O)R₁₁, -NR₁₀C(=O)NR₁₁R₁₂, -NR₁₀C(=O)OR₁₁, -NR₁₀S(=O)₂NR₁₁R₁₂, -OR₁₀, -OC(=O)R₁₀, -OC(=O)OR₁₀, -OC(=O)ONR₁₀R₁₁, -OC(=O)SR₁₀, -C(=O)OR₁₀, -C(=O)R₁₀, -C(=O)NR₁₀R₁₁, -SR₁₀, -S(=O)R₁₀, -S(=O)₂R₁₀, -S(=O)₂NR₁₀R₁₁,
R₁₀, R₁₁ and R₁₂ each represent, independently of each other, a hydrogen atom or a linear or branched (C₁-C₆)-alkyl group, a linear or branched (C₂-C₆)alkenyl group, a linear or branched (C₂-C₆)alkynyl group, a (C₃-C₈)-cycloalkyl or (C₄-C₈)cycloalkenyl group, a heterocyclo-alkyl group containing 3 to 8 atoms in the ring, a (C₅-C₁₁)bicycloalkyl or (C₇-C₁₁)bicycloalkenyl group, a heterobicycloalkyl group containing 5 to 11 atoms in the ring, a bicycloheteroalkenyl group containing 5 to 11 atoms in the ring, or a (C₆-C₁₁)aryl or heteroaryl group containing 5 to 12 atoms in the ring,
R₁₀, R₁₁ and R₁₂ may be optionally substituted, independently of each other, with a halogen atom or a (C₁-C₆)alkyl, (C₁-C₆)alkoxy, nitro, amino, trifluoromethyl or cyano group, or -NR₁₃R₁₄, -NR₁₃C(=O)R₁₉, -NR₁₃C(=O)NR₁₄R₁₅, -NR₁₃C(=O)OR₁₄, -NR₁₃S(=O)₂NR₁₄R₁₅, -OR₁₃, -OC(=O)R₁₃, -OC(=O)OR₁₃, -OC(=O)ONR₁₃R₁₄, -OC(=O)SR₁₃, -C(=O)OR₁₃, -C(=O)R₁₃, -C(=O)NR₁₃R₁₄, -SR₁₃, -S(=O)R₁₃, -S(=O)₂R₁₃, -S(=O)₂NR₁₃R₁₄,
R₁₃, R₁₄ and R₁₅ each represent, independently of each other, a hydrogen atom or a linear or branched (C₁-C₆)-alkyl group, a linear or branched (C₂-C₆)alkenyl group, a linear or branched (C₂-C₆)alkynyl group, a (C₃-C₈)-cycloalkyl or (C₄-C₈)cycloalkenyl group, a heterocyclo-alkyl group containing 3 to 8 atoms in the ring, a (C₅-C₁₁)bicycloalkyl or (C₇-C₁₁)bicycloalkenyl group, a heterobicycloalkyl group containing 5 to 11 atoms in the ring, a bicycloheteroalkenyl group containing 5 to 11 atoms in the ring or a (C₆-C₁₁)aryl or heteroaryl group containing 5 to 12 atoms in the ring, and
R₃ and R₄, or R₄ and R₅, or R₅ and R₆, may together form, with the two carbon atoms that bear them, another aromatic or heteroaromatic ring containing 6 atoms in the ring, optionally substituted with 1 to 4 substituents chosen from those defined for R₇, R₈ and R₉,
in base form or in the form of an addition salt with an acid.

2. Compound according to Claim 1, **characterized in that** X represents a group of general formula C-R₂.

3. Compound according to Claim 2, **characterized in that** P, Q, R and W each represent, respectively, a group of general formula C-R₃, C-R₄, C-R₅ and C-R₆.

4. Compound according to Claim 2, **characterized in that** one of the symbols P, Q, R and W represents a nitrogen atom.

5. Compound according to Claim 1, **characterized in that** X represents a nitrogen atom.

6. Compound according to Claim 5, **characterized in that** P, Q, R and W each represent, respectively, a group of general formula C-R₃, C-R₄, C-R₅ and C-R₆.

7. Compound according to Claim 5, **characterized in that** one of the symbols P, Q, R and W represents a nitrogen atom.

8. Process for preparing a compound according to Claim 1, **characterized in that** 1,4-diazabicyclo[3.2.2]nonane is reacted with a compound of general formula (III) 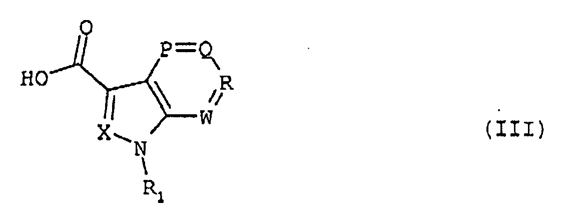 in which X, P, Q, R, W and R₁ are as defined in Claim 1, in the presence of a coupling agent and in a solvent, optionally after a prior step of converting the carboxylic acid function into an acid chloride function.

9. Process for preparing a compound according to Claim 1, **characterized in that** 1,4-diazabicyclo[3.2.2]nonane is reacted with a compound of general formula (IV) 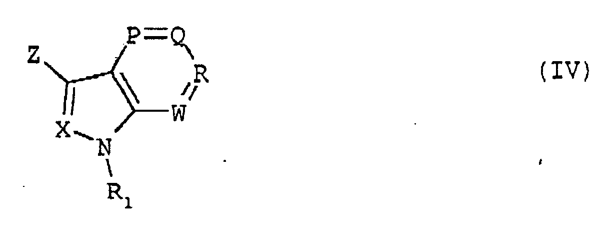 in which X, P, Q, R, W and R₁ are as defined in Claim 1 and Z represents a bromine or iodine atom, in the presence of carbon monoxide, a palladium catalyst and a base, in a solvent.

10. Pharmaceutical composition, **characterized in that** it contains a compound according to Claim 1 and an excipient.

## Patentansprüche

1. Verbindung der allgemeinen Formel (I), 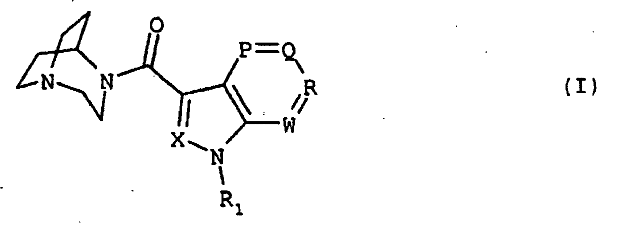 mit den folgenden Bedeutungen:
X bedeutet ein Stickstoffatom oder eine Gruppe der allgemeinen Formel C-R₂;
P bedeutet eine Gruppe der allgemeinen Formel C-R₃;
Q bedeutet eine Gruppe der allgemeinen Formel C-R₄;
R bedeutet eine Gruppe der allgemeinen Formel C-R₅;
W bedeutet eine Gruppe der allgemeinen Formel C-R₆; oder eines der Symbole P, Q, R und W bedeutet ein Stickstoffatom,
R₁ bedeutet ein Wasserstoffatom oder eine (C₁-C₆)Alkylgruppe,
R₂ bedeutet ein Wasserstoffatom oder eine (C₁-C₆)Alkylgruppe,
R₃, R₄, R₅ und R₆ bedeuten jeweils unabhängig voneinander ein Wasserstoff- oder Halogenatom oder eine (C₁-C₆)Alkyl-, (C₁-C₆)Alkoxy-, Nitro-, Amino- Trifluormethyl- oder Cyanogruppe oder eine Gruppe der allgemeinen Formel -NR₇R₈, -NR₇C(=O)R₈, -NR₇C(=O)NR₈R₉, -NR₇C-(=O)OR₈, -NR₇S(=O)₂NR₈R₉, -OR₇, -OC (=O)R₇, -OC(=O)OR₇, -OC(=O)ONR₇R₈, -OC(=O)SR₇, -OS(=O)₂R₇, C(=O)-OR₇, -C(=O)R₇, -C(=O)NR₇R₈, -SR₇, -S(=O)R₇, -S(=O)₂R₇, -S-(=O)₂NR₇R₈, (C₆-C₁₁)Aryl oder (C₃-C₁₂)Heteroaryl, gegebenenfalls durch eine oder mehrere Gruppen aus der Reihe Halogenatome, (C₁-C₆)Alkyl-, (C₁-C₆)Alkoxy-, Nitro-, Amino-, Trifluormethyl-, Cyanogruppen oder Gruppen der allgemeinen Formeln -NR₇R₈ oder -OR₇ substituiert, R₇, R₈ und R₉ bedeuten jeweils unabhängig voneinander ein Wasserstoffatom oder eine geradkettige oder verzweigte (C₁-C₆)Alkylgruppe, eine geradkettige oder verzweigte (C₂-C₆)Alkenylgruppe oder eine geradkettige oder verzweigte (C₂-C₆)Alkinylgruppe oder eine (C₃-C₈)Cycloalkyl-, (C₄-C₈)Cycloalkenylgruppe, oder eine Heterocycloalkylgruppe mit 3 bis 8 Atomen im Ring oder eine (C₅-C₁₁)Bicycloalkyl-, (C₇-C₁₁)Bicycloalkenyl-, Heterobicycloalkylgruppe mit 5 bis 11 Atomen im Ring, eine Bicycloheteroalkenylgruppe mit 5 bis 11 Atomen im Ring, eine (C₆-C₁₁)Arylgruppe oder Heteroarylgruppe mit 5 bis 12 Atomen im Ring, R₇, R₈ und R₉ können gegebenenfalls unabhängig voneinander durch ein Halogenatom oder eine (C₁-C₆)Alkyl-, (C₁-C₆)Alkoxy-, Nitro-, Amino-, Trifluormethyl-, Cyanogruppe oder eine Gruppe der allgemeinen Formel -NR₁₀R₁₁, -NR₁₀C(=O)R₁₁, -NR₁₀C(=O)-NR₁₁R₁₂, -NR₁₀C(=0) OR₁₁, -NR₁₀S(=O)₂NR₁₁R₁₂, -OR₁₀, -OC-(=O)R₁₀, -OC(=0) OR₁₀, -OC(=O)ONR₁₀R₁₁, -OC(=O)SR₁₀, -C (=O)-OR₁₀, -C(=O)R₁₀, -C(=O)NR₁₀R₁₁, -SR₁₀, -S(=O)R₁₀, -S(=O)₂-R₁₀, -S(=O)₂NR₁₀R₁₁ substituiert sein,
R₁₀, R₁₁ und R₁₂ bedeuten jeweils unabhängig voneinander ein Wasserstoffatom oder eine geradkettige oder verzweigte (C₁-C₆)Alkylguppe, eine geradkettige oder verzweigte (C₂-C₆)Alkenylgruppe oder eine geradkettige oder verzweigte (C₂-C₆)Alkinylgruppe oder eine (C₃-C₈)Cycloalkyl-, (C₄-C₈)Cycloalkenylgruppe, oder eine Heterocycloalkylgruppe mit 3 bis 8 Atomen im Ring oder eine (C₅-C₁₁)Bicycloalkyl-, (C₇-C₁₁)Bicycloalkenyl-, Heterobicycloalkylgruppe mit 5 bis 11 Atomen im Ring, eine Bicycloheteroalkenylgruppe mit 5 bis 11 Atomen im Ring, eine (C₆-C₁₁)Arylgruppe oder Heteroarylgruppe mit 5 bis 12 Atomen im Ring,
R₁₀, R₁₁ und R₁₂ können gegebenenfalls unabhängig voneinander durch ein Halogenatom oder eine (C₁-C₆)Alkyl-, (C₁-C₆)Alkoxy-, Nitro-, Amino-, Trifluormethyl-, Cyanogruppe oder eine Gruppe der allgemeinen Formel -NR₁₃R₁₄, -NR₁₃C(=O)R₁₄, -NR₁₃C(=O)NR₁₄R₁₅, -NR₁₃C-(=O)OR₁₄, -NR₁₃S(=O)₂NR₁₄R₁₅, -OR₁₃, -OC(=O)R₁₃, -OC-(=O)OR₁₃, -OC(=O)ONR₁₃R₁₄, -OC(=O)SR₁₃, -C(=O)OR₁₃, -C(=O)-R₁₃, -C(=O)NR₁₃R₁₄, -SR₁₃, -S(=O)R₁₃, -S(=O)₂R₁₃, -S(=O)₂-NR₁₃R₁₄ substituiert sein,
R₁₃, R₁₄ und R₁₅ bedeuten jeweils unabhängig voneinander ein Wasserstoffatom oder eine geradkettige oder verzweigte (C₁-C₆)Alkylgruppe, eine geradkettige oder verzweigte (C₂-C₆)Alkenylgruppe oder eine geradkettige oder verzweigte (C₂-C₆)Alkinylgruppe oder eine (C₃-C₈)Cycloalkyl-, (C₄-C₈)Cycloalkenylgruppe, oder eine Heterocycloalkylgruppe mit 3 bis 8 Atomen im Ring oder eine (C₅-C₁₁)Bicycloalkyl-, (C₇-C₁₁)Bicycloalkenyl-, Heterobicycloalkylgruppe mit 5 bis 11 Atomen im Ring, eine Bicycloheteroalkenylgruppe mit 5 bis 11 Atomen im Ring, eine (C₆-C₁₁)Arylgruppe oder Heteroarylgruppe mit 5 bis 12 Atomen im Ring, und
R₃ und R₄ oder R₄ und R₅ oder R₅ und R₆ können gemeinsam und mit den zwei Kohlenstoffatomen, an die sie gebunden sind, einen weiteren aromatischen oder heteroaromatischen Cyclus mit 6 Atomen im Ring, der gegebenenfalls durch 1 bis 4 Substituenten aus der Reihe der für R₇, R₈ und R₉ definierten Substituenten substituiert ist, bilden,
als Base oder Säureadditionssalz.

2. Verbindung nach Anspruch 1, **dadurch gekennzeichnet, daß** X eine Gruppe der allgemeinen Formel C-R₂ bedeutet.

3. Verbindung nach Anspruch 2, **dadurch gekennzeichnet, daß** P, Q, R und W jeweils eine Gruppe der allgemeinen Formel C-R₃, C-R₄, C-R₅ bzw. C-R₆ bedeuten.

4. Verbindung nach Anspruch 2, **dadurch gekennzeichnet, daß** eines der Symbole P, Q, R und W ein Stickstoffatom bedeutet.

5. Verbindung nach Anspruch 1, **dadurch gekennzeichnet, daß** X ein Stickstoffatom bedeutet.

6. Verbindung nach Anspruch 5, **dadurch gekennzeichnet, daß** P, Q, R und W jeweils eine Gruppe der allgemeinen Formel C-R₃, C-R₄, C-R₅ bzw. C-R₆ bedeuten.

7. Verbindung nach Anspruch 5, **dadurch gekennzeichnet, daß** eines der Symbole P, Q, R und W ein Stickstoffatom bedeutet.

8. Verfahren zur Herstellung einer Verbindung nach Anspruch 1, **dadurch gekennzeichnet, daß** man 1,4-Diazabicyclo[3.2.2]nonan mit einer Verbindung der allgemeinen Formel (III), 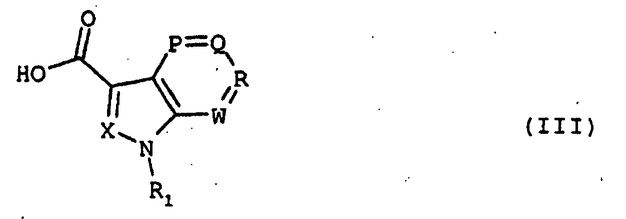 in der X, P, Q, R, W und R₁ wie in Anspruch 1 definiert sind, in Gegenwart eines Kopplungsmittels und in einem Lösungsmittel, gegebenenfalls nach einem vorherigen Schritt, in dem die Carbonsäurefunktion in eine Säurechloridfunktion umgewandelt wurde, umsetzt.

9. Verfahren zur Herstellung einer Verbindung nach Anspruch 1, **dadurch gekennzeichnet, daß** man 1,4-Diazabicyclo[3.2.2]nonan mit einer Verbindung der allgemeinen Formel (IV), 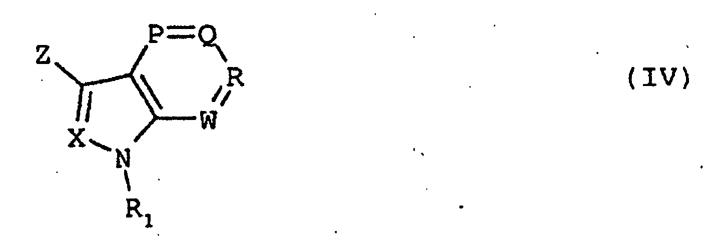 in der X, P, Q, R, W und R₁ wie in Anspruch 1 definiert sind und Z ein Brom- oder Iodatom bedeutet, in Gegenwart von Kohlenmonoxid, eines Palladiumkatalysators und einer Base in einem Lösungsmittel umsetzt.

10. Pharmazeutische Zusammensetzung, **dadurch gekennzeichnet, daß** sie eine Verbindung nach Anspruch 1 und einen Grundstoff enthält.
